**Europäisches Patentamt**

(19) **European Patent Office**

**Office européen des brevets**

(11) Veröffentlichungsnummer: **0 119 511**

**B1**

(12) **EUROPÄISCHE PATENTSCHRIFT**

(45) Veröffentlichungstag der Patentschrift:
**12.08.87**

(51) Int. Cl.⁴: **A 61 M 15/00**

(21) Anmeldenummer: **84101891.4**

(22) Anmeldetag: **23.02.84**

(54) Inhalator mit einem Dampfdruckgefäss zur Wasserdampferzeugung.

(30) Priorität: **12.03.83 DE 3308802**

(43) Veröffentlichungstag der Anmeldung:
**26.09.84 Patentblatt 84/39**

(45) Bekanntmachung des Hinweises auf die Patenterteilung:
**12.08.87 Patentblatt 87/33**

(84) Benannte Vertragsstaaten:
**AT BE CH DE FR GB IT LI LU NL SE**

(56) Entgegenhaltungen:
**CH-A-228 343**
**DE-C-456 526**
**DE-U-7 724 288**
**FR-A-915 784**
**FR-A-2 014 071**

(73) Patentinhaber: **Adolf Ruoff GmbH Kunststoff-Press- und Spritzwerk, Tiefestrasse 4, D-5608 Radevormwald 1 (DE)**

(72) Erfinder: **Erlemann, Bernhard, Wasserturmstrasse 49, D-5608 Radevormwald (DE)**

(74) Vertreter: **Sturies, Herbert, Patentanwälte Dr. Ing. Dipl. Phys. Herbert Sturies Dipl. Ing. Peter Eichler Postfach 20 12 42, D-5600 Wuppertal 2 (DE)**

## Beschreibung

Die Erfindung bezieht sich auf einen Inhalator mit den Merkmalen des Oberbegriffs das Anspruchs 1.

Inhalatoren dienen zur Bekämpfung von Erkrankungen der Atemwege. Bei Erkältungskrankheiten ist es allgemein bekannt, Wasser zu erhitzen, Inhalationsmittel zuzugeben, und die vom Wasserdampf transportierten ätherischen Öle od. dgl. einzuatmen. Dabei wird der Kopf über das dampfende Wasser gehalten und mit einem Handtuch abgedeckt oder es wird ein Topf mit Gesichtsmaske verwendet. In beiden Fällen ergeben sich beträchtliche Unannehmlichkeiten für den Inhalierenden und die Dampfabgabe erfolgt nur kurzzeitig, so daß die mit dieser Inhalation erreichte Wirkung nur gering ist. Es sind auch Inhalatoren bekannt, bei denen der durch Beheizung des Wassers erzeugte Wasserdampf durch Düsen oder mit Ventilatoren zum Inhalierenden befördert wird. Derartige Geräte finden sich vorwiegend in Arztpraxen. Sie sind aufwendig, müssen regelmäßig gewartet werden und sind von dem Inhalierenden normalerweise nicht zu bedienen. Zu solchen Geräten rechnet beispielsweise das Inhaliergerät des DE-U- 77 24 288.

Aus der CH-A- 228 343 ist ein Inhalator mit den eingangs angesprochenen Merkmalen bekannt, bei dem der Kochtopf durch den verwendeten Deckel in einen Bronchitiskessel umgewandelt wird. Dieser dient nicht als Dampfdruckkochtopf zur Zubereitung von Speisen. Die verwendete Sicherheitseinrichtung besteht aus zwei den Deckel an den Topf andrückenden Federn, die bei Dampfdrucküberschreitung eine unkontrollierte Deckelöffnung zulassen, so daß eine Gefährdung des Inhalierenden durch unkontrolliert ausströmenden Dampf möglich ist.

Der Erfindung liegt daher die Aufgabe zugrunde, einen Inhalator der eingangs genannten Art so zu verbessern, daß der Inhalierende zu Hause mit einem einfachen Gerät über längere Zeit inhalieren kann.

Diese Aufgabe wird durch die im kennzeichnenden Teil des Anspruchs 1 aufgeführten Merkmale gelöst.

Im Dampfdruckkochtopf wird Wasserdampf erzeugt, der unter Überdruck steht. Dieser unter Überdruck stehende Wasserdampf wird zur Inhalation ausgenutzt. Die Wärmekapazität ist überlicherweise so groß bzw. der in einem Dampfdruckkochtopf gespeicherte Wasserdampf reicht ohne weiteres aus, um zehn Minuten und länger inhalieren zu können. Die Inhalation kann an einem beliebigen Ort stattfinden. Wird der Dampfdruckkochtopf kontinuierlich erwärmt, z.B. durch eine Heizplatte eines Küchenherdes oder eine sonstige Heizplatte, so kann bis zum vollständigen Verbrauch des in den Dampfdruckkochtopf gefüllten Wassers inhaliert werden. Die Inhalationszeit ist dann praktisch unbegrenzt. Vorteilhafterweise wird also der in einem Dampfdruckkochtopf mit einem Überdruck zwischen 0,75 und 1,08 bar erzeugte Dampf bei der Inhalation ausgenutzt, der den Vorteil hat, in jedem Fall fremdkeimfrei zu sein. Das ist nicht der Fall bei den eingangs beschriebenen Verfahren, bei denen in unkontrollierter Weise Fremdluft zugemischt wird, welche durch den bereits abgekühlten Dampf bzw. das abgekühlte Luft/ Inhalationsmittelgemisch nicht zuverlässig desinfiziert ist. Die an sich erforderliche Desinfektion ist bei dem erfindungsgemäßen Inhalator wesentlich wirksamer, weil die Energie zum Durchströmen des Inhalators im wesentlichen vom Wasserdampf selbst stammt und nicht von einem Gebläse erzeugt werden muß, so daß entsprechend geringere Zuluftmengen benötigt werden, die zuverlässig keimfrei gemacht werden.

Das Anschlußstück der Dampfzuleitung ist lösbar am Deckel befestigt. Infolgedessen kann das Anschlußstück zusammen mit den des weiteren zur Inhalation verwendeten Teilen entfernt und das Dampfdruckgefäß als Dampfdruckkochtopf zum Zubereiten von Speisen verwendet werden, nachdem das im Deckel befindliche Loch für das Anschlußstück durch eine Verschlußvorrichtung verschlossen wurde. Ist bereits ein Dampfdruckkochtopf vorhanden, so braucht lediglich ein zusätzlicher Deckel angeschafft oder umgerüstet zu werden.

Eine Dampfdurchlaßbohrung des Anschlußstücks ist von einem Stellglied ganz oder teilweise absperrbar, das die Dampfdurchlaßbohrung als Verstellschraube mit einem Absperrdorn durchgreift. Infolgedessen ist es ohne weiteres möglich, daß zunächst unter Druck stehender Dampf im Dampfdruckgefäß erzeugt wird, ohne daß Dampf durch den Inhalator ausströmt. Ist genügend Dampf erzeugt bzw. der vorbestimmte Dampfdruck erreicht, so kann das Stellglied betätigt und der Dampf entsprechend herausgelassen werden. Dabei ist es vorteilhaft, daß zunächst geringe Dampfmengen entnommen werden können, damit sich der Inhalierende an die Temperatur gewöhnen kann und eine Verbrennungsgefahr der Schleimhäute ausgeschlossen ist, wie sie bei den herkömmlichen Geräten oft nicht vermieden werden kann. Die Inhalation kann durch Verschliessen der Dampfdurchlaßbohrung mit dem Stellglied auch ohne weiteres unterbrochen werden. Eine derartige Ausgestaltung des Anschlußstücks und seines Stellglieds erleichtern die für Hausgeräte erforderliche einfache konstruktive Ausbildung.

In diesem Sinne ist in die Dampfzuleitung zwischen die Maske und das Anschlußstück des Kochtopfdeckels ein am Anschlußstück befestigter Inhalationsmittelbehälter eingebaut, der eine Inhalationsmittel ansaugende und im Dampf verteilende Düsenvorrichtung aufweist. Die Verwendung von Inhalationsmitteln ergibt eine entsprechende zusätzliche Beeinflussung der erkrankten Bereiche. Auf die Zugabe von Inhalationsmittel kann auch verzichtet werden, so daß der Heilungsprozeß allein durch die Erwärmung beschleunigt wird.

Die Düsenvorrichtung weist eine mit der Dampfdurchlaßbohrung des Anschlußstücks fluchtende Düsenbohrung auf, vor deren Ausgang eine Dampfstrahlprallplatte angeordnet ist, die insbesondere Bestandteil eines auf den Inhaltionsmittelbehälter aufgesteckten Atemausgangsstückes ist. Der Dampf wird also geradewegs durch das Anschlußstück in die Düsenvorrichtung bzw. deren entsprechend ausgerichtete Düsenbohrung geleitet, so daß sich ein entsprechend kurzer Weg und ein entsprechend nur geringer Wärmeverlust ergibt. Es kann eine an sich bekannte Düsenvorrichtung verwendet werden, die innerhalb eines topfförmigen Inhalationsmittelbehälters sitzt, in den das Inhalationsmittel nach seiner Verbindung mit dem Anschlußstück eingegeben wurde.

Das Atemauslaßstück wird in üblicher Weise verwendet, um das Ausatmen zu ermöglichen, ohne daß der Inhalierende das Gesicht von der Maske nehmen muß. Die Verbindung der Dampfstrahlprallplatte mit dem Atemauslaßstück stellt eine Vereinfachung dar, da die Dampfstrahlprallplatte sonst an der Düsenvorrichtung befestigt werden müßte und seine mit dem Atemauslaßstück einstückige Ausbildung kein Problem darstellt.

Die Maske ist an das Atemauslaßstück über ein Verbindungsstück angeschlossen, das S-Form hat und bedarfsweise mit einem Verstellgelenk versehen ist, das eine durchbohrte und ein Teilrohr des Verbindungsrohres aufnehmende Gelenkkugel besitzt, die allseitig beweglich in einer Kugelschale eines zweiten Teilrohrs des Verbindungsrohrs gelagert ist. Das Verbindungsstück ermöglicht es, Abstand zwischen dem Dampfdruckgefäß und der Maske zu schaffen bzw. letztere so anzuordnen, daß eine bequeme Haltung des Inhalierenden möglich ist, unabhängig davon, wo das Dampfdruckgefäß aufgestellt ist. Das Verstellgelenk bietet insoweit eine weitere Bequemlichkeit.

In der Maske oder deren Verbindungsstück zum Teilrohr mit der Kugelschale, zum Atemauslaßstück oder zum Anschlußstück ist ein dampfdurchströmbarer poröser Inhalationsmitteleinsatz angeordnet, der vorzugsweise siebartig ausgebildet ist. In diesen kann Inhalationsmittel eingeträufelt werden, das dann vom durchströmenden Dampf mitgenommen und an die umströmten Bereiche der Atemwege des Körpers transportiert wird. Ein solcher Inhalationsmitteleinsatz ist konstruktiv einfacher und erspart die Verwendung eines Inhalationsmittelbehälters mit Düsenvorrichtung.

Die Erfindung wird anhand von in der Zeichnung dargestellten Ausführungsbeispielen erläutert. Es zeigt:

Fig. 1 eine Schnittdarstellung eines erfindungsgemäßen Inhalators mit Dampfdruckkochtopf, Anschlußstück, Inhalationsmittelbehälter, Atemauslaßstück und Maske,

Fig. 2 eine der Fig. 1 ähnliche Ausbildung eines Inhalators mit einem mit einer Gelenkkugel versehenen Verbindungsrohrs,

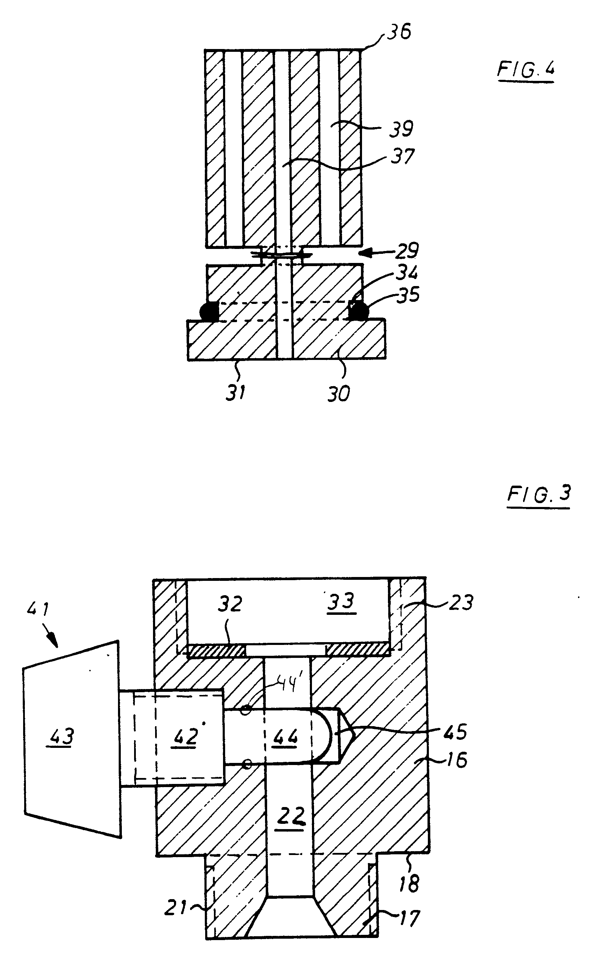

Fig. 3 die konstruktive Ausführung des Verbindungsstücks der Fig. 1, 2,

Fig. 4 eine Düsenvorrichtung,

Fig. 5 eine Ansicht des Verbindungsstücks der Fig. 2 in teilweiser Schnittdarstellung,

Fig. 6 eine Schnittdarstellung der Maske und des Verbindungsstücks der Fig. 2 mit einem im Verbindungsbereich angeordneten Inhalationsmitteleinsatz,

Fig. 7 Einzeldarstellungen dieses Inhalationsmitteleinsatzes,

Fig. 8 eine Schnittdarstellung eines weiteren Verbindungsrohrs,

Fig. 9 eine Aufsicht auf das Verbindungsrohr der Fig. 8 in Richtung A,

Fig. 10 eine Ansicht des Verbindungsrohrs der Fig. 9 in Richtung B und

Fig. 11, 12 je eine Schnittdarstellung des Anschlusses des Inhalators an ein Sicherheitsventil eines Kochtopfdeckels.

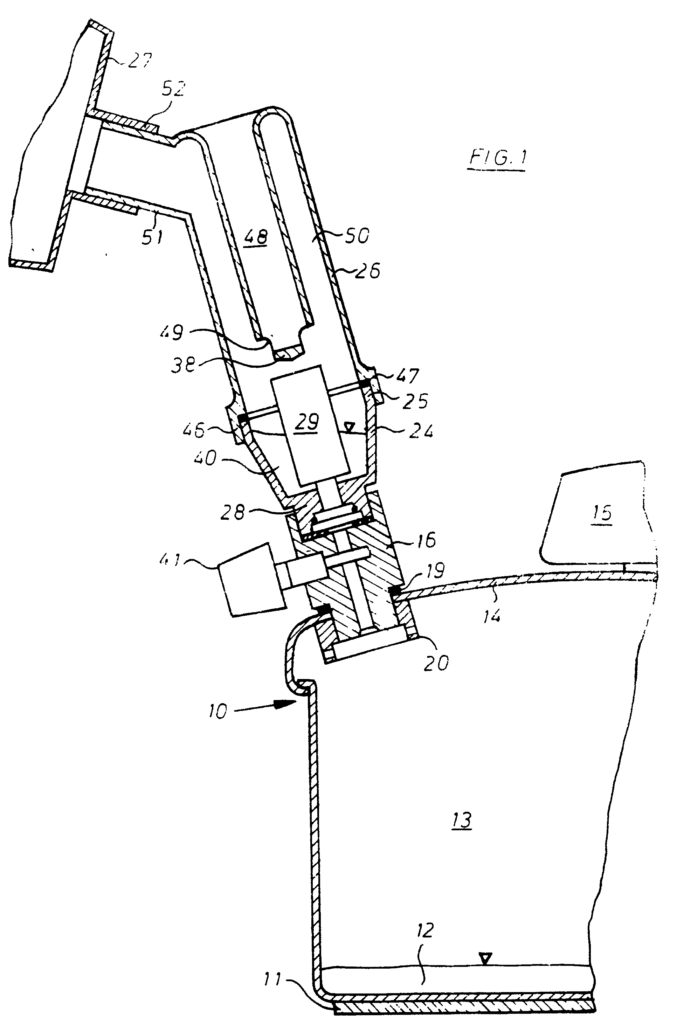

Fig. 1 zeigt einen Dampfdruckkochtopf 10 der über einen Boden 11 erhitzt wird und das Wasser 12 zum Kochen bringt. Im Innenraum 13 des Dampfdruckkochtopfes 10 entwickelt sich daher ein Überdruck, wenn der Topf durch einen Deckel 14 abgeschlossen ist. Der Deckel 14 hat ein Überdruckventil 15, das in üblicher Weise als Sicherheitsventil ausgebildet ist. Bei wachsendem Überdruck wandert ein nicht dargestellter Anzeigestift nach oben aus. Wird der höchstzulässige Druck überschritten, so öffnet das Sicherheitsventil und läßt Dampf ab. Das Ventil 15 gestattet es, den Überdruck im Bereich zwischen 0,75 und 1,08 bar zu regeln.

Am Deckel 14 ist ein Anschlußstück 16 befestigt, das mit einem Anschlußstutzen 17 durch ein nicht bezeichnetes Loch des Deckels 14 gesteckt ist und mit einer Schulter 18 einen Dichtungsring 19 gegen die Oberseite des Deckels 14 preßt, wenn eine Befestigungsmutter 20 auf das Anschlußgewinde 21 des Anschlußstutzens 17 geschraubt wird. Die Verbindung des Anschlußstücks 16 mit dem Dampfdruckkochtopf 10 ist also dicht und lösbar.

Das Anschlußstück 16 hat eine Dampfdurchlaßbohrung 22, die durch den Anschlußstutzen 17 hindurch bis zum Bereich eines Schraubanschlusses 23 geführt ist, mit dem ein Inhalationsmittelbehälter 24 verbunden ist. Der Inhalationsmittelbehälter 24 ist konisch bzw. trichterartig ausgebildet und hat einen Anschlußrand 25, auf den ein Atemauslaßstück 26 gesteckt oder geschraubt ist, das seinerseits eine Maske 27 trägt, die als Gesichtsmaske ausgebildet ist. Der Inhalationsmittelbehälter 24 hat einen Boden 28, mit dem eine Düsenvorrichtung 29 festgehalten wird. Dazu hat die Düsenvorrichtung 29 einen Düsenfuß 30, der mit seiner Fußsohle 31 auf einen Dichtungsring 32 gesetzt wird, der in der Ausnehmung 33 des Anschlußstücks 16 innerhalb des Schraubanschlusses 23 angeordnet ist. Der Düsenfuß 30 hat eine Dichtungsnut 34 mit einem Dichtungsring 35, mit dem eine Abdichtung am Boden 28 des Inhalationsmittelbehälters 24 erreicht wird.

Die Düsenvorrichtung 29 hat einen Düsenkörper 36, der aus dem Inhalationsmittelbehälter 24 bis in das Atemauslaßstück 26 hineinragt. Es ist eine mittige Düsenbohrung 37 vorhanden, die mit der Dampfdurchlaßbohrung 22 fluchtet und sich von der Fußsohle 31 der Düsenvorrichtung 29 bis in die Nähe einer Dampfstrahlprallplatte 38 des Atemauslaßstücks 26 erstreckt. Die Dampfstrahlprallplatte 38 kann auch an dem Düsenstück 36 befestigt sein. Parallel zur Düsenbohrung 37 sind ein oder mehrere Saugbohrungen 39 angeordnet, die in das Inhalationsmittel 40 des Inhalationsmittelbehälters 24 eintauchen. Düsenfuß 30 und Düsenkörper 36 sind in nicht dargestellter Weise zweiteilig ausgebildet, so daß sie z. B. durch gegenseitige Verschraubung mit dem Boden 28 zusammengebaut werden können, der den Düsenfuß 30 beim Einschrauben des Inhalationsmittelbehälters 24 in die Befestigungsvorrichtung 23 des Anschlußstücks 16 gegen den Dichtungsring 33 drückt.

Das Anschlußstück 16 ist mit einem Stellglied 41 versehen, das eine Verstellschraube 42 aufweist, die durch eine Handhabe 43 betätigt wird, beispielsweise einen gerillten Drehknopf. Die Verstellschraube 42 hat einen Absperrdorn 44, der quer zur Dampfdurchlaßbohrung 22 in eine Sackbohrung 45 des Anschlußstücks 16 hineinragt. Der Absperrdorn 44 kann durch Herausdrehen der Verstellschraube 42 die Dampfdurchlaßbohrung 22 ganz oder teilweise freigeben. Auf dem Absperrdorn 44 befindet sich ein Dichtungsring 44'.

Das Atemauslaßstück 26 sitzt mit einem Ringbund 46 aufgesteckt auf dem Anschlußrand 25 des Inhalationsmittelbehälters 24, wobei eine Abdichtung durch einen Dichtungsring 47 erfolgt, die innerhalb des Ringbunds 46 auf der Außenkante des Anschlußrandes 25 angeordnet ist.

Das Atemauslaßstück 26 ist doppelrohrförmig, wobei der Rohrinnenraum 48 über Öffnungen 49 mit dem Rohraußenraum 50 in Verbindung steht, der seinerseits über einen Rohrstutzen 51 in ein Verbindungsstück 52 der Maske 27 eingreift. Der Rohraußenraum 50 ist nach außen abgeschlossen, wohingegen der Rohrinnenraum 48 nach außen offen ist.

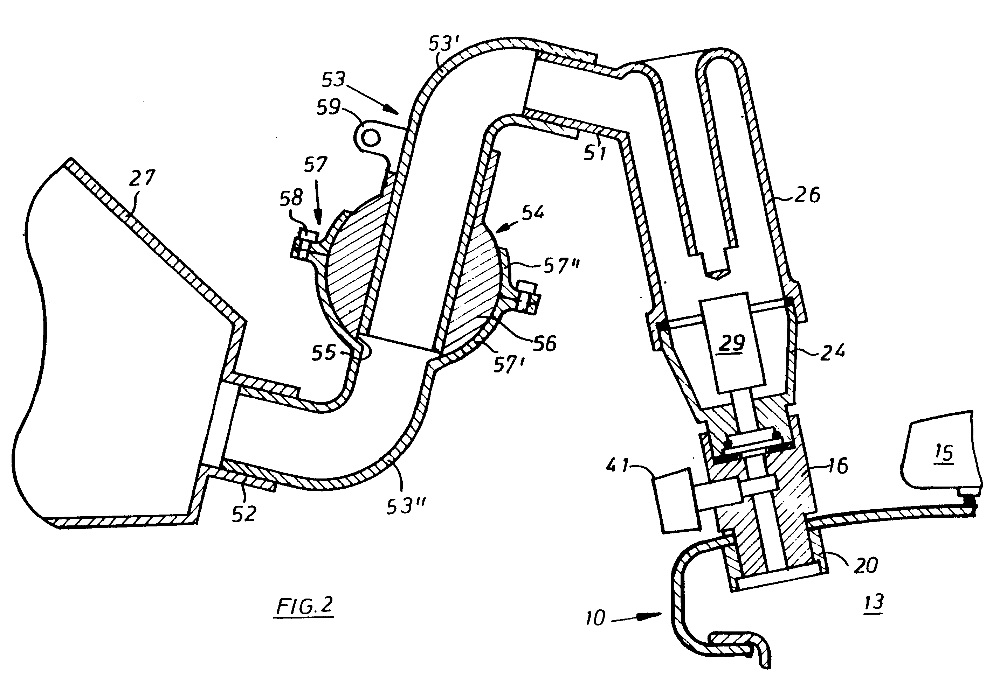

Gemäß Fig. 2 ist zwischen dem Rohrstutzen 51 und der Maske 27 ein Verbindungsrohr 53 angeordnet. Dieses besteht aus Teilrohren 53', 53'', die über ein Kugelgelenk 54 miteinander verbunden sind. Das obere Teilrohr 53' umgreift mit einem Ende den Rohrstutzen 51 und steckt mit seinem anderen Ende in einer Bohrung 55 einer Gelenkkugel 56. Das andere Teilrohr 53'' steckt mit einem Ende in dem Verbindungsstück 52 der Maske 27 und hat an seinem anderen Ende eine Kugellschale 57. Diese besteht aus einer Teilschale 57', die mit dem Teilrohr 53' einstückig ist, und aus einem Ringteil 57'', welches über Verbindungsschrauben 58 mit der Teilschale 57 verbunden ist und die Gelenkkugel 56 so weit umgreift, daß sich eine gelenkige, aber unlösbare Verbindung zwischen den beiden Teilrohren 53', 53'' ergibt. Das Verbindungsrohr 53 bzw. die Gelenkkugel 54 ist mit einer Schraubklemmvorrichtung 59 ausgerüstet, mit der die Relativlage der Maske 27 geändert wird, indem die Gelenkkugel 56 verschoben wird.

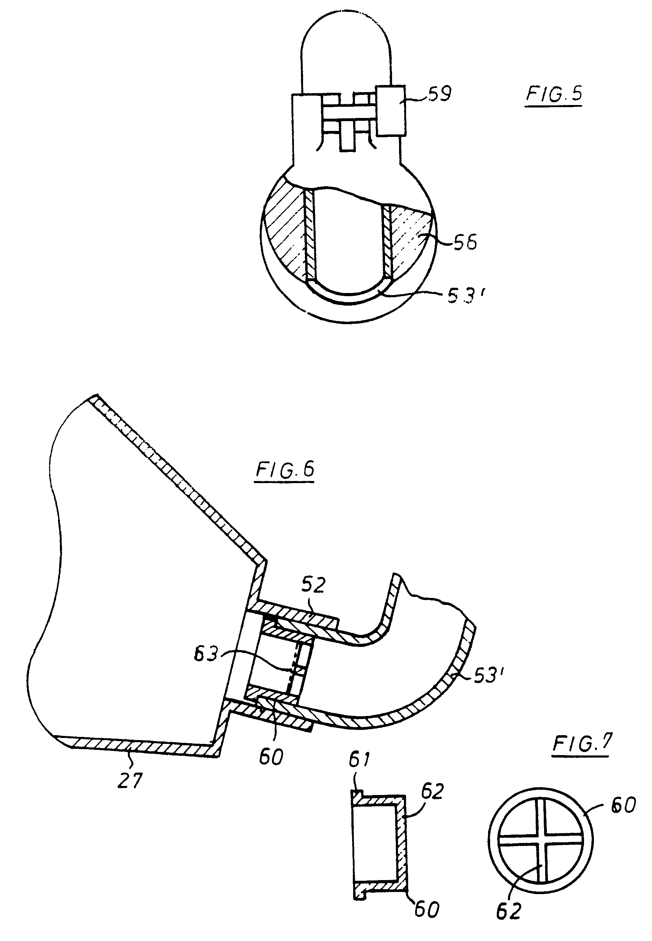

Gemäß Fig. 6 ist in das Verbindungsstück 52 der Maske 27 ein Inhalationsmitteleinsatz 60 eingebaut, der in Fig. 7 näher dargestellt ist. Er ist topfartig und besitzt einen umlaufenden Rand 61, mit dem er auf dem Ende des Teilrohrs 53' aufsitzt. Der Boden 62 ist gemäß der rechten Darstellung in Fig. 7 durchbrochen ausgebildet und gemäß Fig. 6 z. B. mit einer Gaze 63 abgedeckt. Diese Gaze 63 oder eine entsprechende siebartige Ausbildung des Bodens 62 ist infolge ihrer porösen Ausbildung vom Dampf zu durchdringen. Wird vor der Inhalation die Gaze 63 bzw. der siebartige Inhalationsmitteleinsatz mit Inhalationsmittel getränkt, so wird dieses vom durchströmenden Dampf mitgenommen und gelangt in die Atemwege des Inhalierenden.

Es versteht sich, daß Inhalationsmittel auch auf andere Weise in den Inhalator eingegeben werden kann, z. B. durch Zutropfen in einen dampfdurchströmten Bereich des Inhalators.

Inhalation erfolgt, indem der Dampfdruckkochtopf 10 erhitzt wird, so daß sich in seinem Innenraum 13 Dampf entwickelt, der unter Überdruck steht. Wird nun die Verstellschraube 42 so verstellt, daß der Absperrdorn 43 die Dampfdurchlaßbohrung 22 zumindest teilweise freigibt, strömt Dampf durch die Düsenbohrung 37 und prallt auf die Dampfstrahlprallplatte 38, von der aus der Dampfstrahl verwirbelt wird. Dadurch entsteht in den Saugbohrungen 39 ein Unterdruck, der Inhalationsmittel 40 ansaugt, das sich mit dem Dampf vermischt.

Dampf/Luft/Inhalationsmittelgemisch gelangt dann durch das Atemauslaßstück 26 bzw. dessen Rohrstutzen 51 und gegebenenfalls durch das S-förmige Verbindungsrohr 53 in die Maske 27 zum Inhalierenden. Ist der vorbeschriebene Inhalationsmittelbehälter nicht vorhanden, sondern wird stattdessen ein Inhalationsmitteleinsatz 60 verwendet, so strömt der Dampf durch die poröse Gaze 63, nimmt Inhalationsmittel auf und gelangt dann zum Inhalierenden.

Für den Inhalator ist wichtig, daß er im wesentlichen Durchströmwege mit großen Querschnitten besitzt. Lediglich die Dampfdurchlaßbohrung 22 und die Düsenbohrung 37 haben geringere Querschnitte, so daß hier an sich eine Verschmutzung zu befürchten ist. Andererseits wird hier jedoch der infolge seines Überdrucks überhitzte Dampf kurz nach dem Verlassen des Dampfdruckkochtopfes 10 durchgeleitet, so daß eine sofortig Desinfektion auch in dem Fall erfolgt, in dem eine gründliche Reinigung dieser Bohrungen unterblieben ist.

Als Werkstoff für den Inhalator wird im wesentlichen Kunststoff verwendet, abgesehen vom Dampfdruckkochtopf.

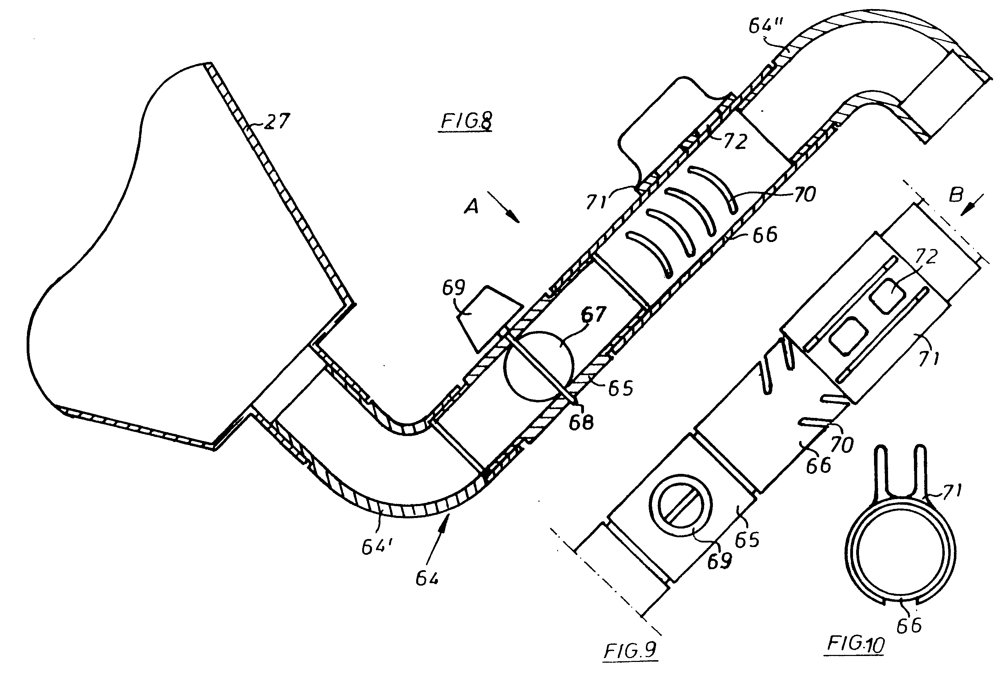

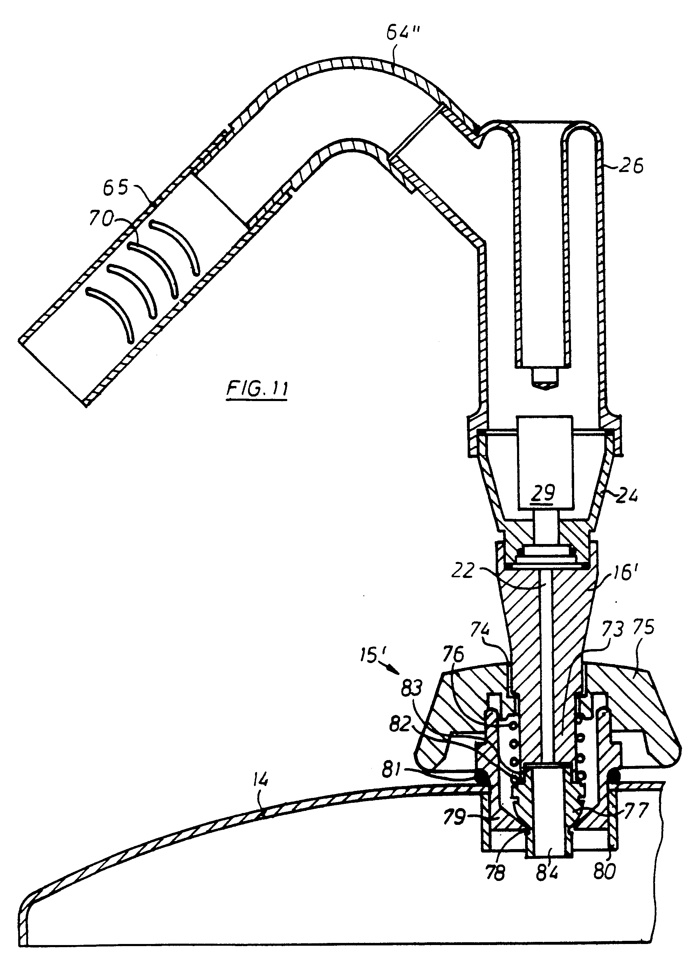

Das in den Fig. 8 bis 10 dargestellte Verbindungsrohr hat zwischen zwei Krümmern 64', 64" angeordnete Rohrstücke 65, 66, die untereinander zusammengesteckt sind. Das Rohrstück 65 ist mit einer Drosselklappe 67 versehen und hat eine Klappenachse 68, an deren einem Ende ein Verstellknopf 69 befestigt ist. Über den Verstellknopf 69 und die Klappenachse 68 kann die Drosselklappe 67 quer zur Strömungsrichtung des Dampfes im Verbindungsrohr 64 gestellt werden und dieses dadurch absperren. Damit kann also die Absperrung auch vom Dampfdruckkochtopf entfernt nahe der Maske 27 vorgenommen werden. Das Rohrstück 66 ist mit Schlitzen 70 versehen, die einen Luftzutritt gestatten, wenn die Schlitze 70 nicht durch einen Schieber 71 verschlossen sind. Je nach Stellung des Schiebers 71 auf dem Rohrstück 66 werden alle oder nur ein Teil der Schlitze 70 verschlossen, um so die Temperatur des in die Maske 27 strömenden Dampfes zu beeinflussen, der je nach Öffnung oder Absperrung der Schlitze 70 mehr oder weniger Luft zusaugt und dementsprechend mehr oder weniger abkühlt. Die Verstellung des Schiebers 71 erfolgt entweder durch Längsverschiebung auf dem Rohrstück 66, oder durch Drehverschiebung, wenn der Schieber 71 auf der die Schlitze 70 aufweisenden Länge des Rohrstücks 66 angeordnet ist. In diesem Fall kann die Zuluft durch den Grad der Überdeckung zwischen den Schlitzen 70 und Schieberöffnungen 72 beeinflußt werden. Durch Verdrehen der Rohrstücke 65, 66 und der Krümmer 64', 64" kann die Vorrichtung für große und kleine Personen leicht eingestellt werden.

Gemäß Fig. 11 wird die nicht näher dargestellte Ventilbohrung des Kochtopfdeckels 14 benutzt, um ein Anschlußstück 16' eines Inhalators anzuschließen. Dabei wird im wesentlichen ein bekanntes Sicherheitsventil 15' benutzt, dessen Anzeigestift jedoch ausgebaut ist. Stattdessen ragt das Anschlußstück 16' mit einem Ende 73 durch eine Bohrung 74 eines Ventildeckels 75, der mit einer Feder 76 auf einen Ventilstück 77 drückt, welcher seinerseits eine Ventilöffnung 78 eines Ventilkörpers 79 verschließt. Der Ventilkörper 79 ist mit dem Ventildeckel 75 in üblicher Weise durch Schraubeingriff verbunden, so daß der Ventildeckel 75 verdreht werden kann. Infolgedessen drückt die Feder 76 mehr oder weniger auf den Ventilkörper 79, so daß der Ventilkörper 77 später oder früher abhebt bzw. der zur Verfügung stehende Dampfdruck größer oder etwas geringer ist. Der Ventilkörper 79 hat an seinem Innenende ein nicht dargestelltes Gewinde zum Aufschrauben einer Befestigungsmutter 80, die beim Einbau des Ventils 15' in den Kochtopfdeckel 14 soweit angezogen wird, daß eine Dichtung 81 abdichtet.

Der Zusammenbau des Anschlußstücks 16' mit dem Ventilstück 77 erfolgt mit einem Umfassungsrand 82, der auf einen Vorsprung 83 des Ventilstücks 77 in nicht dargestellter Weise aufgeschraubt wird. Das Anschlußstück 16' ist im Ventildeckel 75 beweglich geführt, so daß es bei Überdruck im Dampfdruckkochtopf vom Ventilstück 77 nach oben verschoben werden kann, wobei die Ventilöffnung 78 freigegeben wird und der Überdruck entweichen kann.

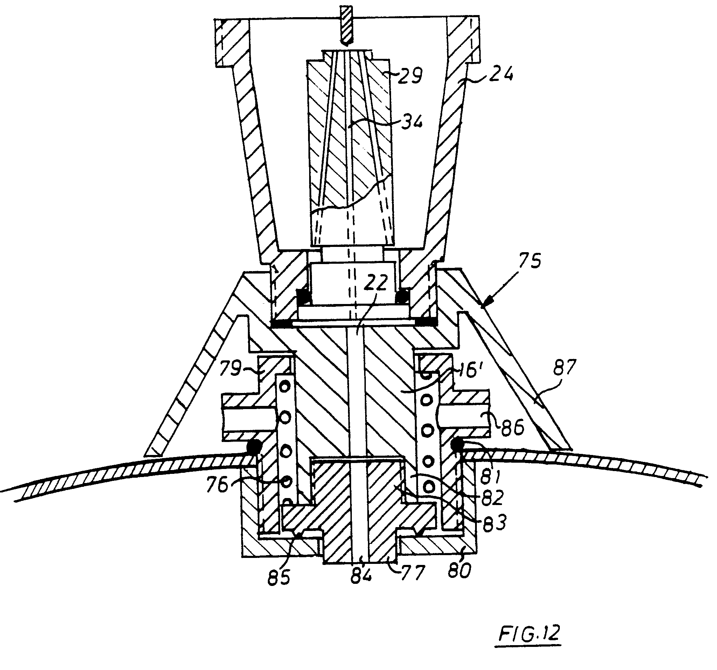

Der Ventildeckel 75 und das Anschlußstück 16' können gemäß Fig. 12 auch einstückig ausgebildet werden. In diesem Falle entfällt der Schraubeingriff zwischen dem Ventildeckel 75 mit dem Ventilkörper 79. Dieser umschließt die Feder 76 und ist am Kochtopfdeckel 14 mit der Dichtung 81 abgedichtet. Zur Abdichtung wird die Befestigungsmutter 80 auf das kochtopfinnenseitige, mit Gewinde versehene Ende des Ventilkörpers 79 aufgeschraubt. Die Befestigungsmutter 80 besitzt innen eine Abdichtungsfläche für das federbelastete Ventilstück 77, das befestigungsmutterseitig mit einem Ringvorsprung 85 versehen ist. Bei Überdruck im Dampfdruckkochtopf hebt sich das Ventilstück 77 entgegen der Kraft der Feder 76 an und Dampf strömt durch das geöffnete Ventil und Überdruckbohrungen 86 unter einen Abdeckkragen 87 des Ventildeckels 75. In den Ventildeckel 75 ist der Inhalationsmittelbehälter 24 eingeschraubt, dessen Düsenvorrichtung 29 für eine Durchmischung des durch die Bohrung 84, die Dampfdurchlaßbohrung 22 und die Düsenbohrung 37 strömenden und sich an der Dampfstrahlprallplatte 38 weiter verteilenden Dampfes mit dem angesaugten Inhalationsmittel sorgt.

Es ist möglich, handelsübliche, mit Sicherheitsventil versehene Kochtopfdeckel 14 zu verwenden, wobei zum Anschluß des Inhalators der Anzeigestift des Sicherheitsventils ausgebaut wird. Zur Vermeidung eines derartigen Umrüstens ist es vorteilhaft, das in Fig. 11 dargestellte Sicherheitsventil 15' mit dem Anschlußstück 16' fabrikmäßig zusammenzubauen und diesen Zusammenbau statt des Sicherheitsventils zu verwenden, der bei normalem Gebrauch des Dampfdruckkochtopfs verwendet wird. Die im Anschlußstück 16' vorgesehene Dampfdurchlaßbohrung 22 fluchtet mit einer Bohrung 84 des Ventilstücks 77. In dieser Bohrung 84 sitzt bei Gebrauch des Sicherheitsventils zur Speisenbereitung der allgemein bekannte Anzeigestift.

**Patentansprüche**

1. Inhalator mit einem Dampfdruckgefäß (10) zur Wasserdampferzeugung bei über dem atmosphärischen Druck liegendem Dampfdruck, mit einem von dem Dampfdruckgefäß abnehmbaren Deckel (14), an dem mit einem Anschlußstück (16, 16') eine zu einer Maske (27) oder einer Düsenvorrichtung (29) führende Inhalationsdampfzuleitung befestigt ist, und mit einer Dampfdruck-Überschreitungen durch Dampfblassen verhindernden Sicherheitseinrichtung, dadurch gekennzeichnet, daß der mit der Inhalationsdampfzuleitung versehene Deckel (14) ein als Sicherheitseinrichtung ein Sicherheitsventil (15, 15') aufweisender Dampfdruckkochtopfdeckel ist.

2. Inhalator nach Anspruch 1, dadurch gekennzeichnet, daß das Anschlußstück (16, 16') der Dampfzuleitung lösbar am Deckel (14) befestigt ist.

3. Inhalator nach Anspruch 1 oder 2, dadurch gekennzeichnet , daß eine Dampfdurchlaßbohrung (22) des Anschlußstücks (16) von einem Stellglied (41) ganz oder teilweise absperrbar ist, das die Dampfdurchlaßbohrung (22) als Verstellschraube (42) mit einem Absperrdorn (44) durchgreift.

4. Inhalator nach einem der Ansprüche 1 bis 3, dadurch gekennzeichnet, daß in die Dampfzuleitung zwischen die Maske (27) und das Anschlußstück (16) des Kochtopfdeckels (14) ein am Anschlußstück (16, 16') befestiger Inhalationsmittelbehälter (24) eingebaut ist, der eine Inhalationsmittel (40) ansaugende und im Dampf verteilende Düsenvorrichtung (29) aufweist.

5. Inhalator nach den Ansprüchen 1 bis 4, dadurch gekennzeichnet, daß die Düsenvorrichtung (29) eine mit der Dampfdurchlaßbohrung (22) des Anschlußstücks (16, 16') fluchtende Düsenbohrung (37) aufweist, vor deren Ausgang eine Dampfstrahlprallplatte (38) angeordnet ist, die insbesondere Bestandteil eines auf den Inhalationsmittelbehälter (24) aufgesteckten Atemauslaßstücks (26) ist.

6. Inhalator nach einem der Ansprüche 4 oder 5, dadurch gekennzeichnet, daß die Maske (27) an das Atemauslaßstück (26) über ein Verbindungsrohr (53, 64) angeschlossen ist, das S-Form hat und bedarfsweise mit einem Verstellgelenk (54) versehen ist, das eine durchbohrte und ein Teilrohr (53') des Verbindungsrohrs (53) aufnehmende Gelenkkugel (56) besitzt, die allseitig beweglich in einer Kugelschale (57) eines zweiten Teilrohrs (53') des Verbindungsrohrs (53) gelagert ist.

7. Inhalator nach Anspruch 6, dadurch gekennzeichnet, daß in der Maske (27) oder deren Verbindungsstück (52) zum Teilrohr mit der Kugelschale (57), zum Atemauslaßstück (26) oder zum Anschlußstück (16) ein dampfdurchströmbarer poröser Inhalationsmitteleinsatz (60) angeordnet ist, der vorzugsweise siebartig ausgebildet ist.

8. Inhalator nach einem der Ansprüche 6 oder 7, dadurch gekennzeichnet, daß das Verbindungsrohr (64) ein mit einer von Hand verstellbaren Drosselklappe (67) versehenes Rohrstück (65) und/oder ein Rohrstück (66) mit Luftzutrittsschlitzen (70) aufweist, die von einem auf diesem Rohrstück (66) sitzenden Schieber (71) abdeckbar sind.

9. Inhalator nach einem der Ansprüche 1 bis 8, dadurch gekennzeichnet, daß das Anschlußstück (16') in eine Ventilbohrung des Sicherheitsventils (15, 15') des Kochtopfdeckels (14) eingebaut ist.

10. Inhalator nach einem der Ansprüche 1 bis 9, dadurch gekennzeichnet, daß das Anschlußstück (16') mit einem federbelasteten Ventilstück (77) des Sicherheitsventils (15') des Kochtopfdeckels (14) zusammengebaut ist, wobei die Dampfdurchlaßbohrung (22) des Anschlußstücks (16') mit einer Bohrung (84) des Ventilstücks (77) fluchtet und/oder daß ein Ventilkörper (79) mindestens eine unter einen Abdeckkragen eines Ventildeckels (75) mündende Überdruckbohrung (86) aufweist und mit einer das federbelastete Ventilstück (77) abstützenden Befestigungsmutter (80) versehen ist.

**Claims**

1. Inhaler with a steam pressure-vessel (10) for producing steam at a pressure above the atmospheric pressure, with a cover (14) removable from the steam pressure-vessel to which an an inhalation steam duct leading to a mask (27) or a nozzle device (29) is secured with a connecting piece (16,16') and with a safety device for preventing steam pressure excesses by vapour escape, characterised in that the cover (14) provided with the inhalation steam duct is a steam pressure heater lid provided as the safety device with a safety valve (15,15').

2. Inhaler according to claim 1, characterised in that the connecting piece (16,16') of the steam duct is detachably secured to the lid (14).

3. Inhaler according to claim 1 or 2, characterised in that a steam access aperture (22) in the connecting piece (16) is partly or wholly closable by a setting member (41), which extends through the steam access aperture (22) as the setting screw (42) with a closure mandrel (44).

4. Inhaler according to any of claims 1 to 3, characterised in that an inhalation medium container (24) secured to the connecting piece (16,16') is provided in the steam duct between the mask (27) and the connecting piece (16) of the heater lid (14) and has a nozzle device (29) extracting an inhalation medium (40) and transmitting it to the steam.

5. Inhaler according to claims 1 to 4, characterised in that the nozzle device (29) includes a nozzle aperture (23) leading to the steam access aperture (22) of the connecting piece (16,16'), before the outlet from which a steam baffle plate (38) is arranged, which in particular is a component of an atomiser connector (26) extending to the inhalation medium container (24).

6. Inhaler according to either of claim 4 or 5, characterised in that the mask (27) is connected to the atomiser connector (26) by means of a connecting tube (53,64), which has an S-form and is provided as required with an adjusting connector (54), which has a through-drilled connecting sphere (56) receiving one part tube (53') of the connecting tube (53), which is mounted fully movably in a spherical shell (57) of a second part tube (53') of the connecting tube (53).

7. Inhaler according to claim 6, characterised in that, in the mask (27) or its connecting member (52) to the part tube with the spherical shell (57), a steam-permeable porous inhalation medium insert (69) is provided for the atomiser connector (26) or for the connecting piece (16), which is preferably made as a screen.

8. Inhaler according to either claim 6 or 7, characterised in that the connecting tube (64) includes a tubular part (65) and/or a tubular part (66) with air-entry slits (70) provided with a manually-adjustable throttle valve (67), which slots are coverable by a slider (71) located on this tubular part (66).

9. Inhaler according to any of claims 1 to 8, characterised in that the connecting piece (16') is constructed in a valve aperture of the safety valve (15,15') of the heater lid (14).

10. Inhaler according to any of claims 1 to 9, characterised in that the connecting piece (16') is constructed with a spring-loaded valve member (77) of the safety valve (15') of the heater lid (14), whereby the steam access aperture (22) of the connecting piece (16') connects with a bore (84) of the valve member (77) and/or that a valve body (79) is provided with at least one overpressure bore (86) discharging beneath a cover projection of a valve cover (75) and provided with a securing nut (80) supporting the spring-loaded valve member (77).

**Revendications**

1. Inhalateur comportant un récipient de vapeur sous pression (10) apte à générer de la vapeur d'eau lorsque la vapeur se trouve à une pression supérieure à la pression atmosphérique, un couvercle (14) séparable du récipient de vapeur sous pression et auquel un conduit de vapeur d'inhalation, aboutissant à un masque (27) ou à un organe d'éjection (29), est fixé par un embout de raccordement (16, 16'), et un dispositif de sécurité empêchant des surpressions de vapeur par un relâchement de vapeur, caractérisé en ce que le couvercle (14), pourvu du conduit de vapeur d'inhalation, est un couvercle d'autoclave comportant, en tant que dispositif de sécurité, une valve de relâchement de pression (15, 15,).

2. Inhalateur selon la revendication 1, caractérisé en ce que l'embout de raccordement (16, 16') du conduit de vapeur est fixé, avec une possibilité de démontage, sur le couvercle (14).

3. Inhalateur selon la revendication 1 ou 2, caractérisé en ce qu'un perçage (22) ménagé dans l'embout de raccordement (16) pour le passage de la vapeur peut être obturé, en totalité ou en partie, par un organe de réglage (41) consistant en une vis (42) à position réglable qui, par un doigt d'obturation (44), pénètre dans le perçage (22) de passage de la vapeur.

4. Inhalateur selon l'une des revendications 1 à 3, caractérisé en ce qu'un réservoir de produit à inhaler (24), fixé à l'embout de raccordement (16, 16'), est inséré dans le conduit de vapeur, entre le masque (27) et l'embout de raccordement (16) du couvercle d'autoclave (14), ce réservoir comportant un organe d'éjection (29) qui aspire le produit à inhaler (40) et le disperse dans la vapeur.

5. Inhalateur selon les revendications 1 à 4, caractérisé en ce que l'organe d'éjection (29) présente un perçage d'éjection (37) qui est aligné avec le perçage (22) ménagé dans l'embout de raccordement (16, 16') pour le passage de la vapeur, et devant la sortie duquel se trouve une plaquette (38) formant une chicane de diffusion de la vapeur, qui est un élément constitutif particulier d'un embout d'échappement de la respiration (26) placé sur le réservoir de produit à inhaler (24).

6. Inhalateur selon l'une des revendications 4 ou 5, caractérisé en ce que le masque (27) est raccordé à l'embout d'échappement de la respiration (26) par un tube de liaison (53, 64) qui a la forme d'un S et est muni, en cas de besoin, d'une articulation de réglage de position (54) possédant une rotule d'articulation (56) qui est traversée de part en part par un perçage pour recevoir un tronçon tubulaire (53') du tube de liaison (53) et qui est montée, en étant mobile dans toutes les directions, dans une coupelle sphérique (57) d'un second tronçon tubulaire (53') du tube de liaison (53).

7. Inhalateur selon la revendication 6, caractérisé en ce que, dans le masque (27) ou dans l'embout (52) qui le relie au tronçon tubulaire muni de la coupelle sphérique (57), à l'embout d'échappement de la respiration (26) ou à l'embout de raccordement (16), est disposé un insert de produit à inhaler poreux (60), apte à être traversé par le flux de vapeur et réalisé de préférence sous la forme d'une grille.

8. Inhalateur selon l'une des revendications 6 ou 7, caractérisé en ce que le tube de liaison (64) comporte une pièce tubulaire (65) munie d'un papillon d'étranglement (67) déplaçable à la main et/ou une pièce tubulaire (66) pourvue de fentes d'admission d'air (70) qui peuvent être recouvertes par un curseur (71) engagé sur cette pièce tubulaire (66).

9. Inhalateur selon l'une des revendications 1 à 8, caractérisé en ce que l'embout de raccordement (16') est inséré dans un perçage de la valve de relâchement de pression (15, 15') du couvercle d'autoclave (14).

10. Inhalateur selon l'une des revendications 1 à 9, caractérisé en ce que l'embout de raccordement (16') est assemblé à un élément de valve (77), chargé par ressort, de la valve de relâchement de pression (15') du couvercle d'autoclave (14), le perçage (22) de passage de la vapeur de l'embout de raccordement (16') étant dans ce cas aligné avec un perçage (84) de l'élément de valve (77) et/ou en ce qu'un corps de valve (79) présente au moins un perçage de surpression (86) débouchant sous une collerette de recouvrement d'un capuchon de valve (75) et est pourvu d'un écrou de fixation (80) soutenant l'élément de valve (77) chargé par ressort.

0 119 511

FIG.1

FIG.2

FIG. 4

36

39

37

29

34

35

31    30

FIG. 3

41

32    33    23

44'

43    42    44    45

16

22

21

18

17

FIG.5

FIG.6

FIG.7

27

*FIG.8*

64"

A

72

71

70

69

66

67

65

68

64'

64

B

72

71

70

66

65

69

*FIG.9*

71

66

*FIG.10*

0119511

FIG. 11

FIG. 12